(19) Europäisches Patentamt
European Patent Office
Office européen des brevets

(11) Veröffentlichungsnummer: **0 312 752 B1**

(12) **EUROPÄISCHE PATENTSCHRIFT**

(45) Veröffentlichungstag der Patentschrift: **18.03.92**

(51) Int. Cl.5: **C07D 207/28**

(21) Anmeldenummer: **88114644.3**

(22) Anmeldetag: **08.09.88**

(54) **Verfahren zur Herstellung von Alkalimetallsalzen der 2-Pyrrolidon-5-carbonsäure.**

(30) Priorität: **17.10.87 DE 3735263**

(43) Veröffentlichungstag der Anmeldung:
**26.04.89 Patentblatt 89/17**

(45) Bekanntmachung des Hinweises auf die Patenterteilung:
**18.03.92 Patentblatt 92/12**

(84) Benannte Vertragsstaaten:
**AT BE CH DE ES FR GB GR IT LI LU NL SE**

(56) Entgegenhaltungen:

**JOURNAL OF THE AMERICAN CHEMICAL SOCIETY, Band 64, Nr. 5, 8. Mai 1942, Seiten 1017-1022; E.H. HUNTRESS et al.: "Identification of organic compounds. VI. The preparation of p-nitrobenzylpyridinium salts of aromatic sulfonic acids", N. LICHTENSTEIN: "Preparation of gamma-alkylamides of glutamic acid"**

**CHEMICAL ABSTRACTS, Band 105, Nr. 13, 29. September 1986, Seite 717, Zusammenfassung Nr. 115402m, Columbus, Ohio, US;**

**CHEMICAL ABSTRACTS, Band 86, Nr. 17, 25. April 1977, Seite 587, Zusammenfassung Nr. 121776s, Columbus, Ohio, US; & JP-A-76 110 559**

(73) Patentinhaber: **Degussa Aktiengesellschaft**
**Weissfrauenstrasse 9**
**W-6000 Frankfurt am Main 1(DE)**

(72) Erfinder: **Drauz, Karlheinz, Dr.**
**Zur Marienruhe 13**
**W-6463 Freigericht 1(DE)**
Erfinder: **Krimmer, Hans-Peter, Dr.**
**Am Weissen Turm 48**
**W-6000 Frankfurt am Main 60(DE)**
Erfinder: **Remmel, Hans**
**Altenmittlauer 44**
**W-6463 Freigericht 3(DE)**

**EP 0 312 752 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein Verfahren zur Herstellung von Alkalimetallsalzen der 2-Pyrrolidon-5-carbonsäure der Formel

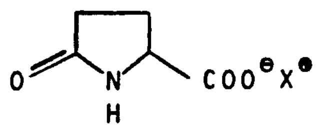

(I),

in der $X^{\oplus}$ ein Alkalimetallion bedeutet.

Die Alkalimetallsalze der 2-Pyrrolidon-5-carbonsäure (Pyroglutaminsäure) sind von vielfältigem Interesse. So ist beispielsweise insbesondere Natrium-pyroglutamat ein weitverbreitetes Agens zur Feuchtigkeitshaltung. Es ist extrem hygroskopisch und vermag innerhalb von 30 Tagen in Luft mit 65 % relativer Luftfeuchtigkeit über 60 % seines eigenen Gewichts an Wasser aufzunehmen. Vor einigen Jahren wurde L-Natrium-pyroglutamat als Bestandteil des "Natürlichen Feuchtigkeitsfaktors" der menschlichen Haut bestimmt [K. Laden, R. Spitzer, J. Soc. Cosmetic Chemists, 18, 351 (1967); [K. Laden, American Perfumer and Cosmetics, 82, 77 (1967)].

Es ist daher nicht verwunderlich, daß Natrium-pyroglutamat heute in zahlreichen kosmetischen Artikeln, wie Hautcremes, Haarwässer, Parfüms, Seifen, Shampoos und Zahnpasten enthalten ist (z.B. JP-OS 50/25741, JP-OS 51/96808, JP-OS 55/49306 oder JP-OS 59/189197). Auch zur Feuchtigkeitshaltung von medizinischen Artikeln, wie Pflastern und Suppositorien, in Tabak, in wasserlöslichen Tinten oder als Zwischenprodukt in der chemischen Synthese, findet Natrium-pyroglutamat Verwendung (z.B. DE-OS 30 23 417).

Die Synthese von L-Natrium-pyroglutamat erwies sich bisher jedoch als relativ umständlich. So wurde die Cyclisierung von einem Gemisch aus L-Glutaminsäure und L-Natriumglutamat in wäßriger Lösung bei 200 °C und erhöhtem Druck beschrieben (JP-OS 51/110559). Nachteilig ist hier die Tatsache, daß man nur eine 50 %ige Lösung des Natrium-pyroglutamats in Wasser erhält, sowie daß eine vollständige Racemisierung zu D,L-Produkt stattfindet, während der natürliche Feuchtigkeitsfaktor lediglich aus L-Natrium-pyroglutamat besteht.

Eine andere Möglichkeit besteht in der Umsetzung von Pyroglutaminsäure mit Natriumhydroxid in Wasser (DE-OS 21 63 939), jedoch entsteht hierbei auch lediglich eine wäßrige Lösung des Salzes. Außerdem wird das Problem verschoben auf die Darstellung der L-Pyroglutaminsäure, die durch Erhitzen von L-Glutaminsäure in konzentrierter wäßriger Lösung nur unter starker Racemisierung und Ausbildung eines Gleichgewichts aus Ausgangsprodukt und Endprodukt gebildet wird. Ein Trennungsschritt ist zur Synthese reiner Pyroglutaminsäure unumgänglich (P.M. Hardy, Synthesis 1978, 290). Auch das direkte Erhitzen von L-Glutaminsäure in der Schmelze auf 180 bis 185 °C ist von Racemisierung und, speziell in diesem Fall, von sehr starker Zersetzung begleitet [N. Lichtenstein, N. Gertner, J. Am. Chem. Soc. 64, 1021 (1946)].

Das erfindungsgemäße Verfahren ist nun dadurch gekennzeichnet, daß man ein Alkalimetallsalz der Glutaminsäure der Formel

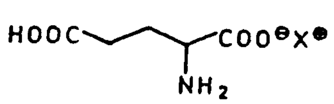

(II),

in der $X^{\oplus}$ wieder das entsprechende Alkalimetallion bedeutet, in Substanz auf eine Temperatur zwischen seinem Schmelzpunkt und 270 °C erhitzt bis das Reaktionswasser der intramolekularen Kondensation vollständig ausgetrieben ist.

Das Aufschmelzen und das Erhitzen des Alkalimetallsalzes der Formel (II) kann sowohl diskontinuierlich als auch kontinuierlich, z. B. in einem Rohrreaktor oder in einer Förderschnecke, erfolgen.

Bei der Durchführung des erfindungsgemäßen Verfahrens wird beispielsweise L-Natriumglutamat in Form seines käuflichen Monohydrats in Substanz, daß heißt in Abwesenheit irgendeines Lösungsmittels oder eines sonstigen flüssigen Wärmeübertragenden Mittels, auf eine Temperatur zwischen 220 und 270

°C erhitzt. Dabei bildet sich eine Schmelze, aus der das Reaktionswasser herausgast. Sobald das Ausgangsmaterial vollständig geschmolzen ist, tritt keine weitere Gasentwicklung mehr auf und das L-Natriumglutamat ist vollständig zu L-Natrium-pyroglutamat umgesetzt. Die Cyclokondensation tritt während des Schmelzvorganges ein. Es ist überhaupt nicht möglich, L-Natriumglutamat zu schmelzen und unverändert zurückzugewinnen.

Bereits bei Temperaturen unterhalb des Schmelz- bzw. Kondensationspunkts von L-Natriumglutamat-monohydrat, der bei ca. 220 °C liegt, tritt eine langsame Abspaltung des Kristallwassers ein. Diese Dehydratisierung ist jedoch sehr langsam. So kann man L-Natriumglutamat-monohydrat bei 180 °C und 0,013 mbar 2 Tage lang tempern und mittels der Karl-Fischer-Methode noch mehr als 50 % des Kristallwassers nachweisen. Eine Cyclisierung zu L-Natrium-pyroglutamat findet jedoch noch nicht statt. Die Dauer des Schmelzvorgangs richtet sich nach der externen Temperatur und der Wärmeübertragungsfähigkeit des Reaktionsgefäßes. Während bei einer Ölbadtemperatur von 220 °C 30 g L-Natriumglutamatmonohydrat ca. 60 Minuten brauchen, um vollständig zu schmelzen, dauert dieser Vorgang bei der gleichen Menge in einem Metallgefäß bei einer Ölbadtemperatur von 270 °C nur eine Minute. Nach dem Abkühlen erstarrt die L-Natrium-pyroglutamatschmelze zu einem farblosen, spröden Glas. Wird die Temperatur nicht für länger als eine Stunde über 250 °C gehalten, oder wird nicht kurzfristig auf eine Temperatur über 270 °C erhitzt, so findet keine Racemisierung oder Bildung von Nebenprodukten statt.

Für analytische oder präparative Zwecke kann man eine verdünnte wäßrige Lösung des L-Natriumpyroglutamats über eine stark saure Ionenaustauschersäule (z.B. Duolite C26) entsalzen und erhält nach Entfernen des Wassers unter vermindertem Druck quantitativ die freie L-Pyroglutaminsäure

Auch die Lithium-, Kalium-, Rubidium- und Caesiumsalze der Glutaminsäure sind als Ausgangsmaterialien für das erfindungsgemäße Verfahren verwendbar, wobei jedes dieser Salze einen charakteristischen Schmelzbereich hat, in dem die Cyclokondensation eintritt. Dieser Schmelzbereich liegt gewöhnlich zwischen 210 und 270 °C.

Die Erfindung wird durch die nachfolgenden Beispiele näher erläutert:

Beispiel 1:

In einem 250 ml Rundkolben wurden 30,0 g (0,160 Mol) L-Natriumglutamat-monohydrat in ein auf 220 °C vorgeheiztes Ölbad getaucht. Nach 5 Minuten begann das Kristallisat zu verkleben und es entwickelte sich Wasserdampf. Es bildete sich langsam eine farblose Schmelze, die heftig gaste. Nach einer Stunde war das Kristallisat vollständig in eine dünnflüssige Schmelze überführt und die Gasentwicklung war beendet. Die Schmelze wurde in eine kalte Porzellanschale gegossen und erstarrte zu einem spröden farblosen Glas. Nach dem Verreiben ergab sich ein farbloses, sehr hygroskopisches Pulver, das bei 125 °C schmolz. Eine HPLC-Analyse [$NH_2$-Säule, 3 µm, 250 x 4,6 mm; Laufmittel: Acetonitril/0,05 M $KH_2PO_4$ (6:4; v/v), UV-Detektor 210 nm] des Produktes zeigte vollständige Umwandlung in L-Natrium-pyroglutamat (Rt = 7,14). L-Natriumglutamat (Rt = 13,42) konnte auch in Spuren nicht mehr detektiert werden. Die Ausbeute betrug 24,0 g (99 % der Theorie).

Zur Überführung in L-Pyroglutaminsäure wurden 10 g des Produktes in 200 ml Wasser gelöst und über eine Ionenaustauschsäule (Duolite C26, $H^+$-Form; 11 cm x 2,5 cm; 30 g) gegeben. Nach dem Eluieren und Entfernen des Wassers unter vermindertem Druck ergaben sich 8,5 g (quantitativ) L-Pyroglutaminsäure vom Schmelzpunkt 155-157 °C [vgl. H. Gibian, E. Klieger, Liebigs Ann. Chem. 640, 145 (1961): Schmelzpunkt: 156-157 °C]. $[\alpha]_D^{25}$ : -11,6 ° (c = 4, $H_2O$) [vgl. A.C. Kibrick, J. Biol. Chem., 174, 845 (1948): $[\alpha]_D^{25}$ : -11,7 ° (c = 4, $H_2O$)] .

Beispiel 2:

In einem unten geschlossenen Stahlzylinder (20 cm x 5 cm), der in ein auf 270 °C vorgeheiztes Ölbad tauchte und dessen Temperatur angenommen hatte, wurden auf einmal 30 g L-Natriumglutamat-monohydrat gegeben. Unter stürmischer Wasserdampfentwicklung schmolz das Ausgangsprodukt innerhalb einer Minute.

Die weitere Aufarbeitung und Charakterisierung erfolgte analog Beispiel 1.

Beispiel 3:

Analog Beispiel 1 wurden 30 g (0,196 Mol) L-Lithiumglutamat bei 270 °C geschmolzen. Es ergaben sich 25,4 g (96 % der Theorie) L-Lithium-pyroglutamat.

Beispiel 4:

Analog Beispiel 1 wurden 30 g (0,162 Mol) L-Kaliumglutamat bei 210 °C geschmolzen. Es ergaben sich 27 g (99 % der Theorie) sehr hygroskopisches L-Kalium-pyroglutamat.

Beispiel 5:

Analog Beispiel 1 wurden 2,0 g (8,64 mMol) L-Rubidiumglutamat bei 210 °C geschmolzen. Es ergaben sich 1,8 g (98 % der Theorie) L-Rubidium-pyroglutamat.

Beispiel 6:

Analog Beispiel 1 wurden 2,0 g (7,17 mMol) L-Caesiumglutamat bei 210 °C geschmolzen. Man erhielt 1,8 g (96 % der Theorie) L-Caesium-pyroglutamat.

## Patentansprüche

1. Verfahren zur Herstellung von Alkalimetallsalzen der 2-Pyrrolidon-5-carbonsäure der Formel

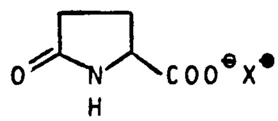

(I),

in der $X^{\oplus}$ ein Alkalimetallion bedeutet, dadurch gekennzeichnet, daß man ein Alkalimetallsalz der Glutaminsäure der Formel

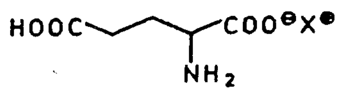

(II),

in der $X^{\oplus}$ wieder das entsprechende Alkalimetallion bedeutet, in Substanz auf eine Temperatur zwischen seinem Schmelzpunkt und 270 °C erhitzt bis das Reaktionswasser der intramolekularen Kondensation vollständig ausgetrieben ist.

2. Verfahren nach Anspruch 1, dadurch gekennzeichnet, daß das Aufschmelzen und das Erhitzen diskontinuierlich erfolgt.

3. Verfahren nach Anspruch 1, dadurch gekennzeichnet, daß das Aufschmelzen und das Erhitzen kontinuierlich erfolgt.

## Claims

1. A process for the preparation of alkali metal salts of 2-pyrrolidone-5-carboxylic acid corresponding to the following formula

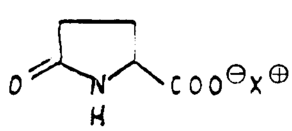

(I),

wherein $X^{(+)}$ denotes an alkali metal ion, characterized in that an alkali metal salt of glutamic acid corresponding to the following formula

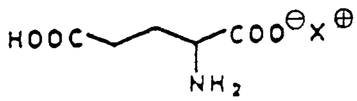

(II),

wherein $X^{(+)}$ again denotes the corresponding alkali metal ion is heated solvent-free to a temperature from its melting point to 270°C until the water of reaction of the intramolecular condensation has been completely driven off.

2. A process according to Claim 1, characterised in that the melting and heating are carried out discontinuously.

3. A process according to Claim 1, characterised in that the melting and the heating are carried continuously.

**Revendications**

1. Procédé d'obtention de sels de métal alcalin de l'acide 2-pyrrolidone-5-carboxylique de formule :

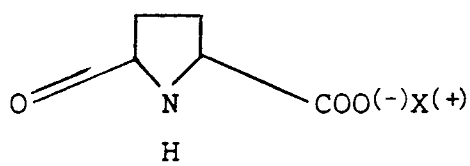

(I)

dans laquelle $X^{(-)}$ signifie un ion de métal alcalin, caractérisé en ce que l'on chauffe un sel de métal alcalin d'acide glutamique, ayant la formule :

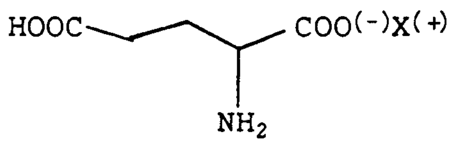

(II)

dans laquelle $X^{(+)}$ à nouveau signifie l'ion de métal alcalin correspondant chauffé, en substance à une température qui s'échelonne entre son point de fusion et 270°C jusqu'à ce que l'eau de réaction de la condensation intra-moléculaire soit complètement chassée.

2. Procédé selon la revendication 1, caractérisé en ce que la fusion et le chauffage sont effectués d'une manière discontinue.

3. Procédé selon la revendication 1, caractérisé en ce que la fusion et le chauffage sont effectués en continu.